# EUROPEAN PATENT SPECIFICATION

(11) **EP 1 427 295 B1**
(45) Date of publication and mention of the grant of the patent: **14.11.2007**
(21) Application number: 02776989.2
(22) Date of filing: 04.09.2002
(51) Int. Cl.: A23L 1/00

(54) **ZEACAROTENE COLORANT FOR FOOD AND PHARMACEUTICALS**
ZEACAROTENE-FARBSTOFF FÜR LEBENSMITTEL ODER PHARMAZEUTIKA
COLORANT POUR PRODUITS ALIMENTAIRES ET PHARMACEUTIQUES

(30) Priority: 13.09.2001 EP 01121981; 04.02.2002 EP 02001968
(43) Date of publication of application: 16.06.2004
(73) Proprietor: DSM IP Assets B.V., 6411 TE Heerlen (NL)
(72) Inventor: BECK, Markus, Ivo, 79540 Loerrach (DE); BERNHARD, Kurt, CH-4419 Lupsingen (CH); GIGER, Alfred, CH-4313 Moehlin (CH); LEUENBERGER, Bruno, CH-4123 Allschwil (CH)
(74) Representative: Müller, Ingrid
(86) International application number: PCT/EP2002/009913
(87) International publication number: WO 2003/022071

(56) References cited:
- US-A- 5 290 605
- US-A- 5 714 658
- US-A- 5 811 609
- US-A- 5 827 539
- US-A- 5 895 659
- DATABASE FSTA [Online] INTERNATIONAL FOOD INFORMATION SERVICE (IFIS), FRANFURT/MAIN, DE; OTLES S, ATLI Y: "Analysis of carotenoids in tomato paste by HPLC and OCC" Database accession no. 2001-00-j1968 XP002234320
- DATABASE BIOSIS [Online] BIOSCIENCES INFORMATION SERVICE, PHILADELPHIA, PA, US; LEE CY, MCCOON PE, LEBOWITZ JM: "Vitamin A value of sweet corn" Database accession no. 198273067950 XP002234321
- DATABASE BIOSIS [Online] BIOSCIENCES INFORMATION SERVICE, PHILADELPHIA, PA, US; GODOY HT, RODRIGUEZ-AMAYA DB: ""BURITI" (MAURITIA VINIFERA MART.), A VERY RICH SOURCE OF PROVITAMIN A" Database accession no. 199598374919 XP002234322

## Description

The present invention concerns a colorant for food and pharmaceuticals. More particularly, the present invention concerns the use of α-zeacarotene and/or β-zeacarotene as a colouring agent for food products or pharmaceutical compositions, colorant compositions containing α-zeacarotene and/or β-zeacarotene as the colouring agent, and food products or pharmaceutical compositions coloured by α-zeacarotene and/or β-zeacarotene.

α-Zeacarotene, which is also known as 7',8'-dihydro-δ-carotene, is a compound of the formula

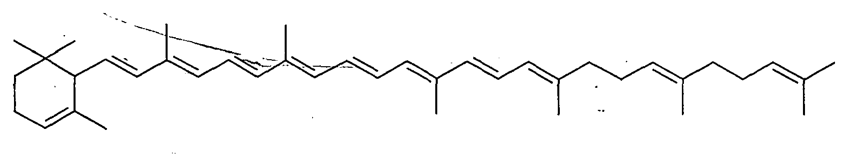

β-Zeacarotene, which is also known as 7',8'-dihydro-γ-carotene, is a compound of the formula

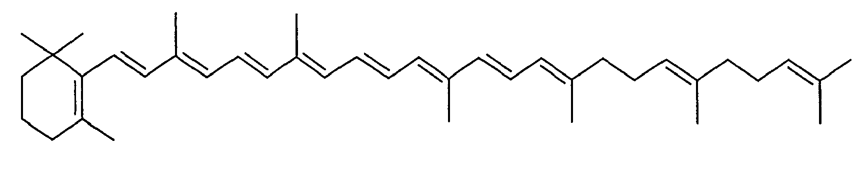

Clearly, these known compounds are structurally very closely related in that the sole difference is the position of the double bond in the cyclohexene ring component.

References for the preparation of each of these compounds are given in Carotenoids, p. 774, O. Isler (ed.), Birkhäuser Verlag Basel and Stuttgart 1971: Chemistry in Britain 3 (10), 424 (1967) and Pure and Appl. Chem. 14, 265 (1967), both by B. C. L. Weedon, and R. Rüegg et al., Helv. Chim. Acta 44, 994 (1961).

In accordance with the present invention it has been found that α-zeacarotene and β-zeacarotene, each individually or a combination of both in any relative proportion (each possibility being hereinafter referred to for simplicity as "the zeacarotene" or the like) can be used to impart a yellow to greenish-yellow colour to food products such as beverages, sweet products and dairy products, and to pharmaceutical compositions, such as tablets and capsules. This use represents one aspect of the present invention. As further aspects, the invention embraces food products or pharmaceutical compositions containing the zeacarotene in an amount sufficient to impart a yellow to greenish-yellow colour to said compositions, and colorant compositions comprising the zeacarotene as the colouring agent, and a matrix or carrier.

For the purpose of the present invention, the zeacarotene is preferably used, i.e. added to the food product or pharmaceutical composition to be coloured or otherwise incorporated in the food product or pharmaceutical composition during its preparation, in the form of a colorant composition, and such a colorant composition is, as mentioned above, embraced by the present invention. The colorant composition containing the zeacarotene may be a solid or a liquid composition. Preferably, the zeacarotene is used as a solid water-dispersible colorant composition. A liquid colorant composition containing the zeacarotene may be a stable aqueous dispersion of the zeacarotene; or it may be a stable suspension of the zeacarotene in a triglyceride, such as a vegetable oil. In order to achieve an intermediate colour hue, such compositions may optionally contain one or more further colouring agents, e.g. one or more further carotenoids such as β-carotene, canthaxanthin, 8'-apo-β-carotenal, ethyl 8' -apo-β-carotenoate, lycopene, astaxanthin, lutein and zeaxanthin.

The colorant compositions of the present invention are preferably solid, pulverous compositions wherein the contained (total) zeacarotene is finely dispersed in a matrix or carrier. In such compositions the amount of the (total) zeacarotene is suitably from about 0.1 to about 30 wt.% based on the total weight of the colorant composition. The matrix or carrier can be any matrix or carrier conventionally used for the formulation of carotenoids. For example, it can be a carbohydrate, a modified carbohydrate, a protein, a modified protein or any mixture of two or more of these.

The preparation of the colorant compositions of the present invention can be carried out in a manner known per se for the preparation of carotenoid and fat soluble vitamin compositions for use in food products, including beverages, e.g. as disclosed in European Patent Publications Nos. 0347751, 0966889, 1066761 and 1106174 and in the PCT Publication No. WO 98/15195, and all such techniques may be employed.

Thus, the compositions of the present invention can be prepared by a process which comprises homogenizing, in an aqueous or colloidal solution of the carrier and optionally one or more water-soluble excipients and/or adjuvants, a solution or dispersion of the zeacarotene and optionally one or more fat-soluble excipients and/or adjuvants in a triglyceride or an organic solvent, or in a mixture of both triglyceride and organic solvent, and, if required, converting the so obtained dispersion into a solid composition. If the dispersion medium for the zeacarotene, i.e. triglyceride, organic solvent or mixture of both, consists predominantly of an organic solvent and contains only a minor amount of triglyceride, the latter may be considered to be present in the role of an excipient and/or adjuvant, in which role it is useful for preventing, or at least considerably retarding, the crystallization of the zeacarotene from the solution, thereby acting as a solvent aid. The whole process may be typically effected as follows:
The carrier and any optionally present water-soluble excipient(s) and/or adjuvant(s) are dissolved in water, affording an aqueous matrix solution. In a separate step the zeacarotene and any optionally present fat-soluble excipient(s) and/or adjuvant(s) are dissolved or suspended in the triglyceride and/or organic solvent. The solution or suspension of the zeacarotene is then added to the aqueous matrix solution, and the mixture is homogenized in order to obtain a fine dispersion of the zeacarotene in the water phase. Finally, if desired, the dispersion is converted into a solid composition.

The process for preparing the colorant composition represents a still further aspect of the present invention.

In the above and further description of the compositions of the present invention containing the zeacarotene as the colouring agent and of their preparation the expression "matrix" means the material environment, except water or another solvent and such functional ingredients as antioxidants and antimicrobial agents, in which the zeacarotene colouring agent is (eventually) dispersed, or more specifically, encapsulated; in many cases it is synonymous with the expression "carrier". In the case of solid water-dispersible forms it is the part of the solid composition which contains the zeacarotene, finely dispersed therein, and which dissolves in water when the composition is added thereto, the zeacarotene then being evenly dispersed in the aqueous medium.

For the homogenization conventional techniques, such as high pressure homogenization, high shear emulsification (rotor-stator systems), micronization or wet milling, can be applied. Other techniques used for the preparation of carotenoid and fat-soluble vitamin compositions for use in food products, including beverages, are disclosed for example in European Patent Publications Nos. 937412 and 1008380 and in US Patent Specification No. 6,093,348, and all such techniques may be employed.

The so obtained dispersion, which is an oil-in-water dispersion, can be converted into a solid composition, e.g. a dry powder, using any conventional technique such as spray drying, spray drying in combination with fluidized bed granulation (the latter technique being commonly known as fluidized spray drying or FSD), or by a powder-catch technique whereby sprayed emulsion droplets are caught in a bed of an absorbent, such as starch, and subsequently dried.

A preferred procedure for preparing a colorant composition of the present invention comprises preparing a solution or dispersion of the zeacarotene and an oil-soluble antioxidant in a triglyceride, e.g. a vegetable oil or fat, and optionally an organic solvent, e.g. a chlorinated hydrocarbon; emulsifying the resulting oil-based solution or dispersion in an aqueous solution prepared from a protective hydrocolloid or carrier such as a protein, a modified protein, a polysaccharide or a modified polysaccharide or any mixture of these, a carbohydrate and, optionally, a water-soluble antioxidant; if required removing the organic solvent, e.g. by evaporation, from the emulsion; drying the emulsion in a manner known per se, e.g. by spraying it into a fluidized starch bed; and finally separating the dried particles, e.g. by sieving.

A further preferred procedure for preparing a colorant composition of the present invention, in this case a suspension of the finely divided zeacarotene in a triglyceride, comprises introducing the zeacarotene and optionally one or more fat-soluble excipients and/or adjuvants in a triglyceride, affording an oil-miscible dispersion of the zeacarotene in the triglyceride, and milling the dispersion, e.g. by ball-milling, thereby converting said dispersion into the desired suspension of finely divided zeacarotene in the triglyceride.

The above-mentioned protective hydrocolloid is a water-soluble polymeric substance acting as the matrix or carrier material and exerting a protective role towards the zeacarotene. As a rule the hydrocolloid features surface activity, by virtue of which it stabilizes emulsions or suspensions in which it is present with the zeacarotene colouring agent. Some, but not all, carriers can act as protective hydrocolloids.

The protective hydrocolloid or carrier which may be present in and used to prepare the colorant compositions of the present invention is for example and more particularly a polysaccharide gum, e.g. gum arabic; a modified food starch, e.g. sodium octenyl succinyl starch; a pectin, e.g. sugar beet pectin; a maltodextrin; a protein, e.g. a gelatin, particularly fish, swine or bovine gelatin, a plant protein or a milk protein; a ligninsulphonate; or any mixture of these substances. Preferably, sodium octenyl succinyl starch or a gelatin is used as the protective hydrocolloid or carrier.

Suitably, the colorant compositions of the present invention (further) contain one or more excipients and/or adjuvants selected from one or more of mono- di-, oligo- and polysaccharides, triglycerides, water-soluble antioxidants and fat-soluble antioxidants. Solid compositions may in addition contain an anti-caking agent, such as silicic acid, and up to about 10 wt.%, as a rule about 2 to about 5 wt.%, of water.

Examples of mono- and disaccharides which may be present in the compositions of the present invention are sucrose, invert sugar, glucose, fructose, lactose, maltose, saccharose and sugar alcohols, and examples of the oligo- and polysaccharides are starch and starch hydrolysates, e.g. dextrins and maltodextrins, especially those having the range of 5 - 65 dextrose equivalents (DE), and glucose syrup, especially such having the range of 20 - 95 DE. The term "dextrose equivalent" (DE) denotes the degree of hydrolysation and is a measure of the amount of reducing sugar calculated as D-glucose based on dry weight; the scale is based on native starch having a DE close to 0 and glucose having a DE of 100.

The triglyceride is suitably a vegetable oil or fat, preferably corn oil, sunflower oil, soybean oil, safflower oil, rape seed oil, peanut oil, palm oil, palm kernel oil, cotton seed oil or coconut oil.

The organic solvent may be for example methylene chloride, chloroform, ethyl acetate, dimethyl ether, acetone, ethanol or isopropanol.

The water-soluble antioxidant may be for example ascorbic acid or a salt thereof, preferably sodium ascorbate. The fat-soluble antioxidant may be for example a tocopherol, e.g. dl-α-tocopherol (i.e. synthetic tocopherol), d-α-tocopherol (i.e. natural tocopherol), β-or γ-tocopherol, or a mixture of two or more of these; butylated hydroxytoluene (BHT); butylated hydroxyanisole (BHA); propyl gallate; tert. butyl hydroxyquinoline; or an ascorbic acid ester of a fatty acid, preferably ascorbyl palmitate or stearate. Depending on the pH of the aqueous matrix solution the ascorbic acid ester of a fatty acid, particularly ascorbyl palmitate or stearate, may alternatively be added to the water phase.

The colorant compositions of the present invention may be solid compositions, i.e. stable, water-soluble or -dispersible powders, or they may be liquid compositions, i.e. aqueous colloidal solutions or oil-in-water dispersions of the aforementioned powders or oil-in-water dispersions of the zeacarotene stabilized by low molecular weight food emulsifiers, said emulsifiers being well known as such. The stabilized oil-in-water dispersions, which may be oil-in-water emulsions or may feature a mixture of suspended, i.e. solid, particles and emulsified, i.e. liquid, droplets, may be prepared by the methods already described above. Other liquid compositions are oil-soluble dispersions of the zeacarotene in triglycerides, e.g. vegetable oils. These oil-soluble dispersions may be physically stabilized by micronization of the dispersed zeacarotene by well-established techniques, such as milling, e.g. with a ball mill or similar means, and/or may be chemically stabilized by adding one or more fat-soluble antioxidants.

Typically, a powder colorant composition according to the present invention comprises
about 0.1 to about 30 wt.%, preferably about 1 to about 20 wt.%, of the zeacarotene;
0 to about 20 wt.%, preferably about 0.5 to about 10 wt.%, of one or more fat-soluble antioxidants;
0 to about 50 wt.%, preferably about 0.5 to about 30 wt.%, of a triglyceride;
about 1 to about 90 wt.%, preferably about 5 to about 70 wt.%, of a protective hydrocolloid;
0 to about 70 wt.%, preferably 0 to about 40 wt.%, of one or more mono- or disaccharides;
0 to about 50 wt.%, preferably 0 to about 35 wt%, of a starch;
0 to about 70 wt.%, preferably 0 to about 40 wt.%, of a starch hydrolysate;
0 to about 10 wt.%, preferably 0 to about 5 wt.%, of a water-soluble antioxidant;
0 to about 5 wt%, preferably about 1 to about 3 wt.%, of silicic acid; and
0 to about 10 wt.%, preferably about 1 to about 5 wt.%, of water,
the weight percentages of all these ingredients totalling 100.

Examples of beverages containing the zeacarotene as an added or incorporated colouring agent and embraced by the present invention are non-alcoholic, flavoured drinks, e.g. flavoured seltzer waters, soft drinks, mineral drinks, flavoured waters, fruit juices, fruit nectars, fruit punches and concentrated forms of these beverages. They may be based on natural fruit or vegetable juices or on artificial juice flavours, and they may be carbonated or non-carbonated. Alcoholic beverages, instant beverage powders, sugar-containing beverages and diet beverages containing non-calorific or artificial sweeteners represent still further examples of beverages which, by virtue of their containing the zeacarotene as a colouring agent, are embraced by the present invention.

Furthermore, dairy products obtained from natural sources are within the scope of the food products in which the zeacarotene is present as an added or incorporated colouring agent, and as such embraced by the present invention. Typical examples of such dairy products are milk drinks, butter, cheese, ice cream, yoghurt and the like. Milk substitute products such as soy milk products and synthetically produced milk substitute products are also included in the food products containing the zeacarotene according to the present invention.

Also included within the scope of the present invention are sweet products containing the zeacarotene as an added or incorporated colouring agent, said sweet products including sugar confectionery products, e.g. boiled sweets, gums, jellies, toffees and fudges, and desserts, including frozen desserts, e.g. sorbets, puddings, instant pudding powders and preserves. Furthermore, fruit preparations used for dairy products and cereals may also be coloured with the zeacarotene, and such coloured fruit preparations are also food products in accordance with the present invention.

Also included within the scope of the present invention are fat-based products, e.g. spreads, including low fat spreads and margarine; low calorific food products containing natural or synthetically produced fat replacers; cereals and cereal products, e.g. cookies, cakes and pasta; and snacks, e.g. d r non-extruded potato-based products, all of which contain the zeacaroten as an added or incorporated colouring agent.

Still further food products which can be coloured with zeacarotene are such miscellaneous products as soups; sauces, e.g. mayonnaise and salad dressings; seasonings; and tofu products; and any compounded foods which contain one or more of the above-mentioned food products.

The concentration of the (total) zeacarotene used as a colouring agent in the food products in accordance with the present invention may be from about 0.1 to about 500 ppm, preferably from about 1 to about 50 ppm, based on the total weight of the food product. Clearly, the concentration range in any particular case depends on the particular food product to be coloured and on the intended grade of colouration in such food product.

If the zeacarotene is used as a colorant composition, the content of the (total) zeacarotene in such a colorant composition may be from about 0.1 wt.% to about 30 wt.%, particularly from about 1 to about 20 wt.%, based on the total weight of the colorant composition, the most suitable (narrower) content range depending on the particular nature of the composition, i.e. on which other ingredients are present therein and on its physical form.

The coloured food products or pharmaceutical compositions of this invention are preferably obtained by adding to or incorporating in the food product or pharmaceutical composition, at a suitable stage in its manufacture, the zeacarotene colouring agent in the form of a colorant composition of this invention. For such colouration of a food product or a pharmaceutical composition the colorant composition of this invention can be used according to methods per se known for the application of water- or oil-dispersible solid or liquid carotenoid forms to food products or pharmaceutical compositions, as appropriate.

For the colouration of a food product the zeacarotene colorant composition may in general be added either as an aqueous stock solution, a dry powder mix or a pre-blend with other suitable food ingredients according to the specific application. Mixing can be effected for example using a dry powder blender, a low shear mixer, a high pressure homogenizer or a high shear mixer, depending on the required nature of the final food product. The particular mixing procedure and amount of oily or aqueous ingredients may influence the colour of the final food product. As will be readily apparent, such technicalities are within the skill of the expert in the art of food manufacture and formulation.

Pharmaceutical compositions such as tablets or capsules in which the zeacarotene is used as a colouring agent are also within the scope of the present invention. The colouration of tablets can be accomplished by adding the zeacarotene in form of a liquid or solid colorant composition separately to the tablet coating mixture or by adding a colorant composition comprising the zeacarotene to one of the components of the tablet coating mixture. Coloured hard or soft shell capsules can be prepared by incorporating a colorant composition comprising the zeacarotene in the aqueous solution of the capsule mass.

In order to obtain a specific intermediate colour hue of the final food product or pharmaceutical composition other than the greenish-yellow hue obtained by using a colorant composition of the zeacarotene alone (α-zeacarotene, β-zeacarotene or a mixture of both), colorant compositions may be used that optionally contain one or more other colouring agents besides the zeacarotene, such as one or more further carotenoids. Alternatively, the zeacarotene colorant composition may be used in combination with other colouring agents as such or contained in appropriate further colorant compositions.

Of the two closely related colouring agents the subject of the present invention, β-zeacarotene is generally preferred over α-zeacarotene.

The following Examples illustrate the invention further:

### Example 1

### Preparation of a colorant composition

### a) Preparation of (oil-based) solution A

2.4 g of corn oil and 1.1 g of dl-α-tocopherol were introduced into a 250 ml Erlenmeyer flask. The flask was transferred to a glove box flushed with nitrogen. Under inert atmosphere, 5.4 g of crystalline β-zeacarotene and, subsequently, 85 ml of methylene chloride were added to the dl-α-tocopherol in the flask, which was then equipped with a magnetic stirrer and a reflux condenser. By gently stirring and at the same time heating the mixture to 40°C a clear solution was obtained within 30 minutes.

### b) Preparation of (aqueous) solution B

A dry premix of 19 g of fish gelatin (Norland Products, Inc.), 38 g of sucrose and 2.4 g of ascorbyl palmitate was prepared and subsequently mixed with 120 g of deionized water at room temperature in a 1l reaction vessel. The mixture was heated to 35°C and stirred for 30 minutes at that temperature. By dropwise addition of a 5N sodium hydroxide solution, the pH of the aqueous solution was maintained at about 8.0.

### c) Preparation of emulsion from solutions A and B

Solution A was added to solution B at 35°C with vigorous stirring and the dispersion was vigorously stirred for another 30 minutes. Residual methylene chloride was removed at 50-55°C using a rotary evaporator. After removing entrapped air bubbles by centrifugation the emulsion was gently stirred at 50-55°C for 15 minutes and then characterized with respect to the particle size of the inner phase. The mean particle size of the inner phase of the emulsion was below 200 nm as measured by photon correlation spectroscopy (Coulter N4 Plus).

### d) Preparation of solid composition from the emulsion

The emulsion was sprayed into a pre-cooled fluidized bed of corn starch. Excess corn starch was removed by sieving and the powder obtained was dried in an air stream at room temperature for about 2 hours. The powder particle fraction in the range of 0.16-0.50 mm (approx. 95 g) was collected by sieving and characterized with respect to the carotenoid content, colour intensity and colour hue in the aqueous dispersion, the content of corn starch and the residual humidity.

The so obtained powder had a β-zeacarotene content of 3.5 wt.% and 3.8 wt.% as measured by UV/VIS spectroscopy and HPLC analysis, respectively. According to HPLC analysis the all-E isomer content was in the range of 80 to 90%. The powder had a content of 33 wt.% corn starch and a residual water content of 6.5 wt.%. The powder was then dispersed in deionized water and the extinction was measured in a 1 cm quartz precision cell against deionized water. For a 10 ppm dispersion of β-zeacarotene an extinction of 1.64 at a wavelength of 438 nm was calculated [E(1%,1 cm) = 1640]. The colour values L*=93.6, a*=-6.9 and b*=66.0 were measured according to the CIE system for a 5 ppm dispersion of β-zeacarotene with a Hunterlab Ultrascan Spectrocolorimeter (1 cm, TTRAN). Based upon the values of a* and b* a colour hue angle h*=96° at a saturation c*=66.4 can be calculated. A 2 ppm β-zeacarotene dispersion of the powder showed a colour hue angle of almost 100°. (A colour hue angle in the range of 90-180° designates a greenish-yellow colour hue).

### Example 2

### Preparation of a colorant composition

### a) Preparation of (oil-based) solution A

5.4 g of a middle chain triglyceride (Bergabest MCT-Oil 60/40 of Berg & Schmidt) and 1.1 g of dl-α-tocopherol were introduced into a 200 ml three-necked reaction flask. The flask was transferred to a glove box flushed with nitrogen. Under inert atmosphere, 5.4 g of crystalline β-zeacarotene were added. The suspension was gently stirred with a magnetic stirred and at the same time heated to 170°C. A clear mixture was obtained within 90 seconds at that temperature. After cooling the mixture to about 50°C, 70 ml of chloroform were added and a clear solution was obtained by heating the mixture to about 65°C for 5 minutes under reflux.

### b) Preparation of (aqueous) solution B

A dry premix of 18 g of fish gelatin (Norland Products, Inc.), 36 g of sucrose and 2.4 g of ascorbyl palmitate was prepared and subsequently mixed with 110 g of deionized water in a 1l reaction vessel. The mixture was heated to 35°C and stirred for 30 minutes at that temperature. By dropwise addition of a 5N sodium hydroxide solution the pH of the aqueous solution was mantained at about 8.0.

### c) Preparation of emulsion from solutions A and B

Solution A was added to solution B at 35°C with vigorous stirring and the dispersion was vigorously stirred for another 30 minutes. Residual chloroform was removed at 50-55°C using a rotary evaporator. After removing entrapped air bubbles by centrifugation the emulsion was gently stirred at 50-55°C for 15 minutes and then characterized with respect to the particle size of the inner phase. The mean particle size of the inner phase of the emulsion was below 250 nm as measured by photon correlation spectroscopy (Coulter N4 Plus).

### d) Preparation of solid composition from the emulsion

The emulsion was sprayed into a pre-cooled fluidized bed of corn starch. Excess corn starch was removed by sieving and the powder obtained was dried in an air stream at room temperature for about 2 hours. The powder particle fraction in the range of 0.16-0.63 mm (approx. 65 g) was collected by sieving and characterized with respect to the carotenoid content, colour intensity and colour hue in the aqueous dispersion, the content of corn starch and the residual humidity.

The so obtained powder had a β-zeacarotene content of 4.1 wt.% and 4.4 wt.% as measured by UV/VIS spectroscopy and HPLC analysis, respectively. According to HPLC analysis the all-E isomer content was in the range of 50 to 55%. The powder had a content of 27 wt.% corn starch and a residual water content of 7.0 wt.%. The powder was then dispersed in deionized water and the extinction was measured in a 1 cm quartz precision cell against deionized water. For a 10 ppm dispersion of β-zeacarotene an extinction of 1.75 at a wavelength of 436 nm was calculated [E(1%,1 cm) = 1750]. The colour values L*=92.8, a*=-7.6 and b*=67.2 were measured according to the CIE system for a 5 ppm dispersion of β-zeacarotene with a Hunterlab Ultrascan Spectrocolorimeter (1 cm, TTRAN). Based upon the values of a* and b* a colour hue angle h*=96.5° at a saturation c*=67.6 can be calculated. A 2 ppm β-zeacarotene dispersion of the powder showed a colour hue angle of 100.5°.

### Example 3

### Soft drink formulation

| Formulation ingredients | Weight in g |
|---|---|
| Sugar syrup 64°Brix | 156.2 |
| Sodium benzoate | 0.2 |
| Ascorbic acid | 0.2 |
| Citric acid (as a 50% w/w aqueous solution) | 5.0 |
| Pectin (as a 2% w/w aqueous solution) * | 10.0 |
| Flavour ** | 0.5 |
| β-Zeacarotene 5% as a 1% stock solution *** | 8.0 |
| Water | to 200.0 |

| | |
|---|---|
| * GENU Pectin Type VIS of Copenhagen Pectin A/S ** Apricot Flavour No. 78511-76 of Givaudan *** A 1 % aqueous solution of a formulation as obtained in Example 1 or 2, said formulation having a β-zeacarotene content of 5 wt.% | |

### Procedure

The sodium benzoate is dissolved in part of the water, and the sugar syrup, ascorbic acid, citric acid, pectin solution and flavour, and finally the β-zeacarotene stock solution, are added. The bottling syrup is diluted to give one litre of beverage.

The β-zeacarotene provides a vivid greenish-yellow colour of a high clarity and with a high colouring strength. Long-time stability tests lasting three months at ambient conditions showed a good light stability of β-zeacarotene in such a beverage and a good colour stability in respect of the colour values L*C*h*.

### Example 4

A jelly, e.g. jelly bears, can be formulated as follows:

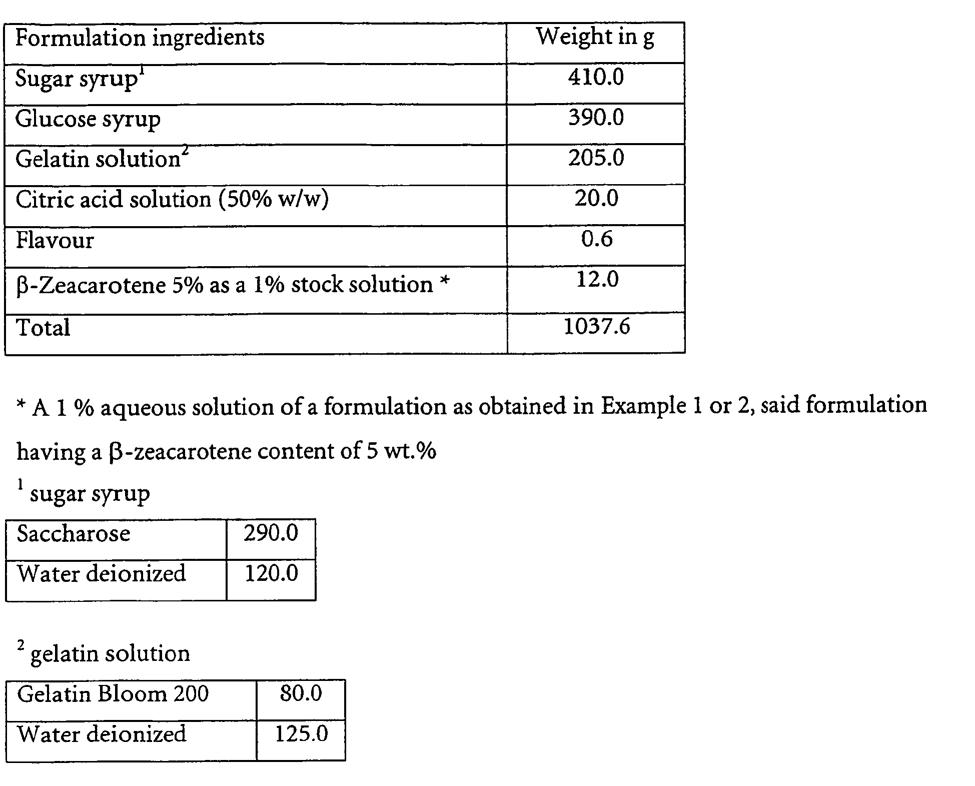

### Procedure

The sugar syrup is prepared by mixing the saccharose with the deionized water and brought to the boil until the solution becomes clear, and the heat source is then removed. To prepare the gelatin solution the gelatin (Bloom 200) is dispersed in cold deionized water, stirred and dissolved by heating. Then the glucose syrup is mixed with the hot sugar syrup without boiling, and the gelatin solution added slowly thereto, avoiding foaming. The mixture is allowed to stand until foam on the surface can be removed and a temperature of 60 to 65°C has been reached. The flavour, citric acid and β-zeacarotene solution are added, and immediately thereafter the mixture is filled into moulds set into starch trays, allowed to set and dried for at least 48 hours under ambient conditions.

Subsequently the starch powder is removed after drying with air (4-6 bar), the moulded product polished with oil or wax or the product surface briefly treated with steam passed from a kettle via a tube, and the resulting jelly bears are transferred to a sugar tumbler. Finally they are dried under ambient conditions and packaged in sealed boxes or pouches.

### Example 5

A milk drink can be formulated as follows:

| Formulation ingredients | Weight in g |
|---|---|
| Sugar, fine granular | 25.0 |
| Dextrose | 20.0 |
| Stabilizer | 0.2 |
| Flavour | 0.8 |
| β-Zeacarotene 5% * | 0.08 |
| Milk 1.5% fat | to 1000.0 |

| | |
|---|---|
| * Solid formulation containing 5 wt.% β-zeacarotene | |

### Procedure

All the dry ingredients, including the β-zeacarotene, are blended, and the dry mixture is dissolved in the milk by heating to 65°C with stirring, The flavour is added and the whole heated to 80°C. Homogenization is then effected in a high pressure homogenizer (p₁ 150 bar, p₂ 50 bar) at 80°C. The resulting UHT milk drink is then passed through a plate heat exchanger with direct steam injection at 140°C for 5 seconds.

The so produced milk drink is packaged by filling into sterilized containers such as laminated board packs or plastic bottles.

### Example 6

### Preparation of a colorant composition

### a) Preparation of (oil-based) solution A

2.4 g of corn oil and 1.1 g of dl-α-tocopherol were introduced into a 250 ml Erlenmeyer flask. 8.8 g of crystalline β-zeacarotene were dissolved in 86 ml of methylene chloride and the solution obtained was added to the corn oil and the tocopherol in the flask, which was then equipped with a magnetic stirrer and a reflux condenser. By gently stirring and at the same time heating the mixture to 40°C a solution was obtained.

### b) Preparation of (aqueous) solution B

A dry premix of 31.2 g of fish gelatin (Norland Products Inc.), 55.2 g of sucrose, 55.2 g of maltodextrin (DE. 20-23, Roquette Frères) and 4.0 g of ascorbyl palmitate was prepared and subsequently mixed with 180 ml of deionized water in a 1l reaction vessel at room temperature. The mixture was heated to 35°C and stirred for about 30 minutes at that temperature. By dropwise addition of a 5N sodium hydroxide solution the pH of the solution was brought to 8.0.

### c) Preparation of emulsion from solutions A and B

Solution A was added to solution B at 35°C with vigorous stirring and the dispersion was vigorously stirred for another 30 minutes. Residual methylene chloride was removed at 55°C using a rotary evaporator. Subsequently, the dispersion was diluted with deionized water at 55°C to a final water concentration of about 65% and then characterized with respect to the particle size of the inner phase. The mean particle size of the inner phase of the emulsion was 172 nm, as measured by photon correlation spectroscopy (Coulter N4 Plus).

### d) Preparation of solid composition from the emulsion

The dispersion was then spray dried using a Mobile Minor (NiroA/S). The inlet temperature of the drying air was 190°C and the outlet temperature was about 78°C. The powder was collected and about 0.5% of silicon dioxide was added. The powder was then characterized with respect to the carotenoid content, colour intensity and colour hue in the aqueous dispersion, the content of corn starch and the residual humidity.

The so obtained powder had a β-zeacarotene content of 5.1 wt.% and 5.6 wt.% as measured by UV/VIS spectroscopy and HPLC analysis, respectively, and a residual water content of 3.5 wt.%. According to HPLC analysis the all-E isomer content was in the range of 80-90%. The powder was dispersed in deionized water and the extinction was measured in a 1 cm quartz precision cell against deionized water. For a 10 ppm dispersion of β-zeacarotene an extinction of 1.601 at a wavelength of 438 nm was calculated [E(1%,1 cm) = 1601]. The colour values L*=93.9, a*=- 6.5 and b*=65.2 were measured according to the CIE system for a 5 ppm dispersion of β-zeacarotene with a Hunterlab Ultrascan Spectrocolorimeter (1 cm, TTRAN). Based upon the values of a* and b* a colour hue angle h*=95.7° at a saturation (chroma value) C*=65.5 can be calculated.

### Example 7

### Preparation of a colorant composition

### a) Preparation of (oil-based) solution A

4.7 g of corn oil and 0.9 g of dl-α-tocopherol were introduced into a 100 ml Erlenmeyer flask. 8.5 g of crystalline α-zeacarotene were dissolved in 110 ml of methylene chloride and the solution obtained was added to the corn oil and the tocopherol in the flask, which was then equipped with a magnetic stirrer and a reflux condenser. By gently stirring and, at the same time, heating the mixture to 40°C a solution was obtained. Minor amounts of insoluble material were removed by filtration.

### b) Preparation of (aqueous) solution B

A dry premix of 37.8 g of bovine gelatin (30 Bloom), 37.8 g of sucrose and 11.2 g of ascorbyl palmitate was prepared and subsequently mixed with 170 ml of deionized water at room temperature in a 1l reaction vessel. The mixture was heated to 40°C and stirred for about 30 minutes at that temperature. By dropwise addition of a 5N sodium hydroxide solution the pH of the solution was brought to 7.5.

### c) Preparation of emulsion from solutions A and B

Solution A was added to solution B at 35°C with vigorous stirring and the dispersion was vigorously stirred for another 30 minutes. After addition of 60 ml of deionized water the reaction vessel was flushed with nitrogen and the dispersion was heated to 55°C. The stirred dispersion was maintained at 55°C for 60 minutes in order to evaporate residual methylene chloride. After removing entrapped air bubbles by centrifugation the emulsion was gently stirred at 50-55°C for 15 minutes and then characterized with respect to the particle size of the inner phase. The mean particle size of the inner phase of the emulsion was 212 nm, as measured by photon correlation spectroscopy (Coulter N4 Plus).

### d) Preparation of solid composition from the emulsion

The emulsion was sprayed into a pre-cooled fluidized bed of corn starch. Excess corn starch was removed by sieving and the powder obtained was dried in an air stream at room temperature for about 2 hours. The powder particle fraction in the range of 0.16-0.50 mm (approx. 165 g) was collected by sieving and characterized with respect to the carotenoid content, colour intensity and colour hue in the aqueous dispersion, the content of corn starch and the residual humidity.

The so obtained powder had an α-zeacarotene content of 3.3 wt.%, as measured by UV/VIS spectroscopy, a content of 56 wt.% corn starch and a residual water content of 4.9 wt.%. It was dispersed in deionized water and the extinction was measured in a 1 cm quartz precision cell against deionized water. For a 10 ppm dispersion of α-zeacarotene an extinction of 1.513 at a wavelength of 431 nm was calculated [E(1%,1 cm) = 1513]. The colour values L*=97.4, a*=-13.1 and b*=48.5 were measured according to the CIE system for a 5 ppm dispersion of α-zeacarotene with a Hunterlab Ultrascan Spectrocolorimeter (1 cm, TTRAN). Based upon the values of a* and b* a colour hue angle h*=105° at a saturation (chroma value) C*=50.2 can be calculated (A colour hue angle in the range of 90-180°C designates a greenish-yellow colour hue).

### Example 8

### Preparation of a colorant composition

### a) Preparation of (oil-based) solution A

3.8 g of corn oil and 1.0 g of dl-α-tocopherol were introduced into a 100 ml Erlenmeyer flask. 8.5 g of crystalline α-zeacarotene were dissolved in 120 ml of methylene chloride and the solution obtained was added to the corn oil and the tocopherol in the flask, which was then equipped with a magnetic stirrer and a reflux condenser. By gently stirring and, at the same time, heating the mixture to 40°C a solution was obtained. Minor amounts of insoluble material were removed by filtration.

### b) Preparation of (aqueous) solution B

A dry premix of 20.3 g of fish gelatin (Norland Products Inc.), 42.1 g of sucrose and 5.7 g of ascorbyl palmitate was prepared and subsequently mixed with 128 ml of deionized water at room temperature in a 1 l reaction vessel. The mixture was heated to 40°C and stirred for about 30 minutes at that temperature. By dropwise addition of a 5N sodium hydroxide solution the pH of the solution was brought to 7.5.

### c) Preparation of emulsion from solutions A and B

Solution A was added to solution B at 35°C with vigorous stirring and the dispersion was vigorously stirred for another 30 minutes. After addition of 100 ml of deionized water the reaction vessel was flushed with nitrogen and the dispersion was heated to 55°C. The stirred dispersion was maintained at 55°C for 60 minutes in order to evaporate residual methylene chloride. After removing entrapped air bubbles by centrifugation the emulsion was gently stirred at 50-55°C for 15 minutes and then characterized with respect to the particle size of the inner phase. The mean particle size of the inner phase of the emulsion was 186 nm, as measured by photon correlation spectroscopy (Coulter N4 Plus).

### d) Preparation of solid composition from the emulsion

The emulsion was sprayed into a pre-cooled fluidized bed of corn starch. Excess corn starch was removed by sieving and the powder obtained was dried in an air stream at room temperature for about 2 hours. The powder particle fraction in the range of 0.16-0.50 mm (approx. 165 g) was collected by sieving and characterized with respect to the carotenoid content, colour intensity and colour hue in the aqueous dispersion, the content of corn starch and the residual humidity.

The so obtained powder had an α-zeacarotene content of 3.9 wt.%, as measured by UV/VIS spectroscopy, a content of 56 wt.% corn starch and a residual water content of 5.3 wt.%. It was dispersed in deionized water and the extinction was measured in a 1 cm quartz precision cell against deionized water. For a 10 ppm dispersion of α-zeacarotene an extinction of 1.418 at a wavelength of 431 nm was calculated [E(1%,1 cm) = 1418]. The colour values L*=97.0, a*=-12.3 and b*=48.7 were measured according the CIE system for a 5 ppm dispersion of α-zeacarotene with a Hunterlab Ultrascan Spectrocolorimeter (1 cm, TTRAN). Based upon the values of a* and b* a colour hue angle h*=104° at a saturation (chroma value) C*=50.2 can be calculated (A colour hue angle in the range of 90-180° designates a greenish-yellow colour hue).

### Example 9

### Soft drink formulation

| Formulation ingredients | Weight in g |
|---|---|
| Sugar syrup, 64°Brix | 156.2 |
| Sodium benzoate | 0.2 |
| Ascorbic acid | 0.2 |
| Citric acid (as a 50% w/w aqueous solution) | 5.0 |
| Pectin (as a 2% w/w aqueous solution) * | 10.0 |
| Flavour ** | 0.5 |
| α-Zeacarotene 5% as a 1% stock solution *** | 8.0 |
| Water | to 200.0 |

| | |
|---|---|
| * GENU Pectin Type VIS of Copenhagen Pectin A/S ** Apricot Flavour No. 78511-76 of Givaudan *** A 1% aqueous solution of a formulation as obtained in Example 8, said formulation having an α-zeacarotene content of 5 wt.% | |

### Procedure

The sodium benzoate is dissolved in part of the water, and the sugar syrup, ascorbic acid, citric acid, pectin solution and flavour, and finally the α-zeacarotene stock solution, are added. The bottling syrup is diluted with water to give one litre of beverage.

The α-zeacarotene provides a vivid greenish-yellow colour of high clarity and with a high colouring strength. Long-time stability tests lasting three months at ambient conditions showed a good light stability of α-zeacarotene in such a beverage and a good colour stability in respect of the colour values L*C*h*.

### Example 10

A jelly, e.g. jelly bears, can be formulated as follows:

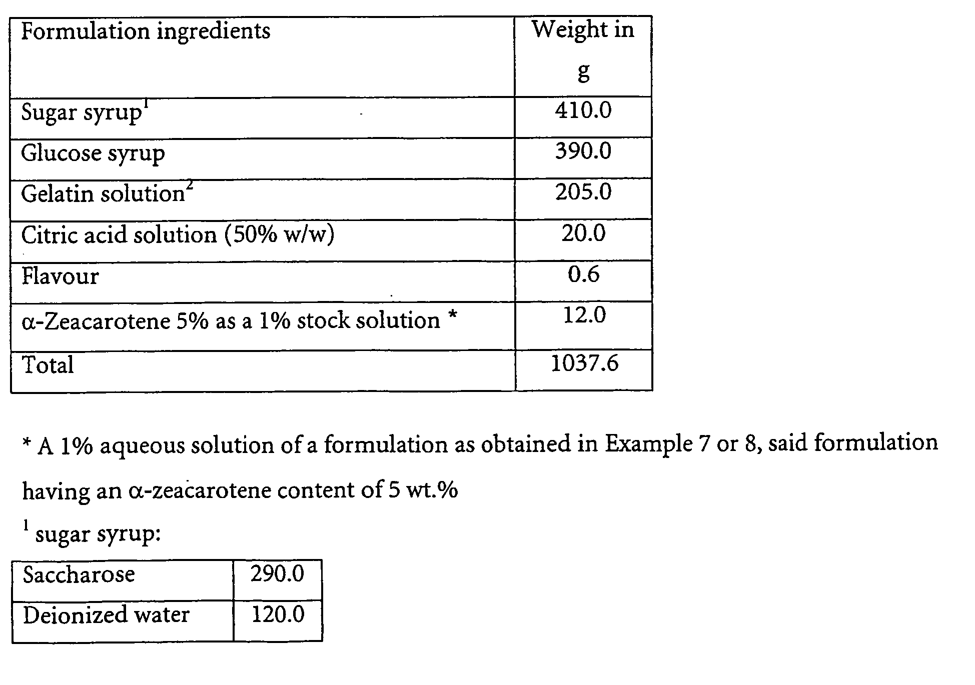

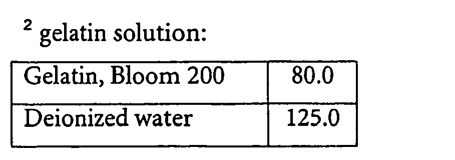

### Procedure

The sugar syrup is prepared by mixing the saccharose with the deionized water and brought to the boil until the solution becomes clear, and the heat source is then removed. To prepare the gelatin solution the gelatin (Bloom 200) is dispersed in cold deionized water, stirred and dissolved by heating. Then the glucose syrup is mixed with the hot sugar syrup without boiling, and the gelatin solution added slowly thereto, avoiding foaming. The mixture is allowed to stand until foam on the surface can be removed and a temperature of 60 to 65°C has been reached. The flavour, citric acid and α-zeacarotene solution are added, and immediately thereafter the mixture is filled into moulds set into starch trays, allowed to set and dried for at least 48 hours under ambient conditions. Subsequently the starch powder is removed after drying with air (4-6 bar), the moulded product polished with oil or wax or the product surface is briefly treated with steam passed from a kettle via a tube, and the resulting jelly bears are transferred to a sugar tumbler. Finally they are dried under ambient conditions and packaged in sealed boxes or pouches.

### Example 11

A milk drink can be formulated as follows:

| Formulation ingredients | Weight in g |
|---|---|
| Sugar, fine granular | 25.0 |
| Dextrose | 20.0 |
| Stabilizer | 0.2 |
| Flavour | 0.8 |
| α-Zeacarotene 5% * | 0.08 |
| Milk 1.5% fat | to 1000.0 |

| | |
|---|---|
| * Solid formulation containing 5 wt.% α-zeacarotene | |

### Procedure

All the dry ingredients, including the α-zeacarotene, are blended, and the dry mixture is dissolved in the milk by heating to 65°C with stirring, The flavour is added and the whole heated to 80°C. Homogenization is then effected in a high pressure homogenizer (p₁ 150 bar, p₂ 50 bar) at 80°C. The resulting UHT milk drink is then passed through a plate heat exchanger with direct steam injection at 140°C for 5 seconds.

The so produced milk drink is packaged by filling into sterilized containers such as laminated board packs or plastic bottles.

## Claims

1. The use of α-zeacarotene or β-zeacarotene or a mixture of α- and β-zeacarotene in any relative proportion as a colouring agent for food products or pharmaceutical compositions.

2. The use according to claim 1, wherein the colouring agent is α-zeacarotene.

3. The use according to claim 1, wherein the colouring agent is β-zeacarotene.

4. The use according to any one of claims 1 to 3, wherein the food products are beverages, dairy products, milk substitute products, sweet products, fruit preparations, fat-based products and low calorific food products, cereals and cereal products, snacks, soups, sauces, seasonings, tofu products and compounded foods which contain one or more of the above-mentioned food products, and the pharmaceutical compositions are tablets or capsules.

5. The use according to any one of claims 1 to 4, wherein the concentration of the α-zeacarotene , the β-zeacarotene or the mixture of α- and β-zeacarotene used as the colouring agent in the food product is from about 0.1 to about 500 ppm, preferably from about 1 to about 50 ppm, based on the total weight of the food product.

6. The use according to any one of claims 1 to 5, wherein the α-zeacarotene, the β-zeacarotene or the mixture of α- and β-zeacarotene has been added to the food product or pharmaceutical composition to be coloured, or otherwise incorporated in the food product or pharmaceutical composition during its preparation, in the form of a colorant composition which is a stable water-soluble or -dispersible powder containing the zeacarotene or zeacarotene mixture, an aqueous colloidal solution or oil-in-water dispersion of the aforementioned powder, an oil-in-water dispersion the zeacarotene or zeacarotene mixture, or an oil-soluble dispersion of the zeacarotene or zeacarotene mixture in a triglyceride.

7. A colorant composition for food products or pharmaceutical compositions, **characterized in that** it contains α-zeacarotene or β-zeacarotene or a mixture of α- and β-zeacarotene in any relative proportion as the colouring agent.

8. A colorant composition according to claim 7, which is a stable water-soluble or -dispersible powder containing the zeacarotene or zeacarotene mixture, an aqueous colloidal solution or oil-in-water dispersion of the aforementioned powder, an oil-in-water dispersion of the zeacarotene or zeacarotene mixture, or an oil-soluble dispersion of the zeacarotene or zeacarotene mixture in a triglyceride.

9. A colorant composition according to claim 8, which is a stable water-soluble or -dispersible powder, the amount of the zeacarotene or zeacarotene mixture being from about 0.1 to about 30 wt.% based on the total weight of the colorant composition.

10. A colorant composition according to claim 9, wherein the zeacarotene or zeacarotene mixture is finely dispersed in a matrix or carrier, preferably a matrix or carrier comprising one or more of a carbohydrate, a modified carbohydrate, a protein, a modified protein or any mixture of two or more of these.

11. A colorant composition according to claim 10, wherein the matrix or carrier, of which at least a part can be a protective colloid, is selected from a polysaccharide gum, a modified food starch, a pectin, a maltodextrin, a protein, a ligninsulphonate and any mixture of these substances.

12. A colorant composition according to claim 11, wherein the matrix or carrier is selected from sodium octenyl succinyl starch as the modified food starch and a gelatin as the protein.

13. A colorant composition according to any one of claims 7 to 12, wherein the composition, in solid or liquid form as the case may be, (further) contains one or more excipients and/or adjuvants selected from one or more each of mono- di-, oligo- and polysaccharides, triglycerides, water-soluble antioxidants, fat-soluble antioxidants and, solely in the case of solid compositions, additionally an anti-caking agent.

14. A colorant composition according to claim 13, wherein the mono- di-, oligo- and polysaccharides are selected from sucrose, invert sugar, glucose, fructose, lactose, maltose, a sugar alcohol, starch, a starch hydrolysate and glucose syrup.

15. A colorant composition according to claim 14, wherein the starch hydrolysate is a dextrin or a maltodextrin having the range of 5 - 65 dextrose equivalents, and the glucose syrup has the range of 20 - 95 dextrose equivalents.

16. A colorant composition according to any one of claims 8 and 13 to 15, wherein the triglyceride is a vegetable oil or fat, preferably corn oil, sunflower oil, soybean oil, safflower oil, rape seed oil, peanut oil, palm oil, palm kernel oil, cotton seed oil or coconut oil.

17. A colorant composition according to any one of claims 13 to 16, wherein the water-soluble antioxidant is ascorbic acid or a salt thereof, preferably sodium ascorbate, and the fat-soluble antioxidant is a tocopherol, butylated hydroxytoluene; butylated hydroxyanisole; propyl gallate; tert. butyl hydroxyquinoline; or an ascorbic acid ester of a fatty acid, preferably ascorbyl palmitate or stearate.

18. A colorant composition according to any one of claims 13 to 17, wherein the anti-caking agent is silicic acid.

19. A process for preparing a colorant composition for food products or pharmaceutical compositions as claimed in any one of claims 7 to 8, **characterized by** homogenizing, in an aqueous or colloidal solution of a carrier and optionally one or more water-soluble excipients and/or adjuvants, a solution or dispersion of the zeacarotene or zeacarotene mixture and optionally one or more fat-soluble excipients and/or adjuvants in a triglyceride or an organic solvent, or in a mixture of both triglyceride and organic solvent, and, if required, converting the so obtained dispersion into a solid composition.

20. A process according to claim 19, **characterized by** preparing a solution or dispersion of the zeacarotene or zeacarotene mixture and an oil-soluble antioxidant in a triglyceride and optionally, an organic solvent, emulsifying the resulting oil-based solution or dispersion in an aqueous solution prepared from a protective hydrocolloid or carrier, a carbohydrate and, optionally, a water-soluble antioxidant; if required removing the organic solvent from the emulsion, drying the emulsion in a manner known per se, and finally separating the dried particles.

21. A process for preparing a colorant composition for food products or pharmaceutical compositions as claimed in any one of claims 7, 8 and 13 to 17 and which is a suspension of finely divided the zeacarotene or zeacarotene mixture in a triglyceride, **characterized by** introducing the zeacarotene or zeacarotene mixture and optionally one or more fat-soluble excipients and/or adjuvants in a triglyceride, affording an oil-miscible dispersion of the zeacarotene or zeacarotene mixture in the triglyceride, and milling the dispersion, e.g by ball-milling, thereby converting said dispersion into the desired suspension of finely divided zeacarotene or zeacarotene mixture in the triglyceride.

22. A food product or pharmaceutical composition, especially a beverage, a dairy product, a milk substitute product, a sweet product, a fruit preparation, a fat-based product, a low calorific food product, a cereal, a cereal product, a snack, a soup, a sauce, seasoning, a tofu product or a compounded food which contain one or more of the above-mentioned food products, or, respectively, tablets or capsules, **characterized by** comprising α-zeacarotene or β-zeacarotene or a mixture of α- and β-zeacarotene in any relative proportion as an added or incorporated colouring agent in an amount sufficient to impart a yellow to greenish-yellow colour to the food product or pharmaceutical composition.

23. A food product according to claim 22, wherein the concentration of the zeacarotene or zeacarotene mixture used as the colouring agent is from about 0.1 to about 500 ppm, preferably from about 1 to about 50 ppm, based on the total weight of the food product.

## Patentansprüche

1. Verwendung von α-Zeacarotin oder β-Zeacarotin oder einem Gemisch aus α- und β-Zeacarotin in jedem beliebigen Verhältnis als Farbstoff für Nahrungsmittelprodukte oder pharmazeutische Zusammensetzungen.

2. Verwendung nach Anspruch 1, wobei der Farbstoff-α-zeacarotin ist.

3. Verwendung nach Anspruch 1, wobei der Farbstoff β-zeacarotin ist.

4. Verwendung nach einem der Ansprüche 1 bis 3, wobei die Nahrungsprodukte Getränke, Milchprodukte, Milchersatzprodukte, Süßigkeiten, Fruchtzubereitungen, Produkte auf Fettbasis und Produkte mit niedrigem Brennwert, Cerealien und Cerealienprodukte, Snacks, Suppen, Soßen, Gewürze, Tofuprodukte und zusammengesetzte Nahrungsmittel sind, die eines oder mehrere der oben genannten Nahrungsmittelprodukte enthalten, und die pharmazeutischen Zusammensetzungen Tabletten oder Kapseln sind.

5. Verwendung nach einem der Ansprüche 1 bis 4, wobei die Konzentration von α-zeacarotin, β-zeacarotin oder dem Gemisch von α- und β-Zeacarotin, das als Farbstoff in dem Nahrungsmittelprodukt verwendet wird, von etwa 0,1 bis etwa 500 ppm, vorzugsweise von etwa 1 bis etwa 50 ppm, bezogen auf das Gesamtgewicht des Nahrungsmittelprodukts, beträgt.

6. Verwendung nach einem der Ansprüche 1 bis 5, wobei α-Zeacarotin, β-Zeacarotin oder das Gemisch von α- und β-Zeacarotin in Form einer Farbstoffzusammensetzung, die ein stabiles wasserlösliches oder -dispergierbares Pulver, welches Zeacarotin oder Zeacarotingemisch enthält, eine wässrige kolloidale Lösung oder eine Öl-in-Wasser-Dispersion des genannten Pulvers, eine Öl-in-Wasser-Dispersion von Zeacarotin oder Zeacarotingemisch oder eine öllösliche Dispersion von Zeacarotin oder Zeacarotingemisch in einem Triglycerid ist, zu dem zu färbenden Nahrungsmittelprodukt oder der zu färbenden pharmazeutischen Zusammensetzung gegeben wird oder anderweitig in das Nahrungsmittelprodukt oder die pharmazeutische Zusammensetzung während seiner/ihrer Herstellung eingebracht wird.

7. Farbstoffzusammensetzung für Nahrungsmittelprodukte oder pharmazeutische Zusammensetzungen, **dadurch gekennzeichnet, dass** sie α-zeacarotin oder β-Zeacarotin oder ein Gemisch aus α- und β-Zeacarotin in jedem beliebigen Verhältnis als Farbstoff enthält.

8. Farbstoffzusammensetzung nach Anspruch 7, die ein stabiles wasserlösliches oder -dispergierbares Pulver, welches Zeacarotin oder Zeacarotingemisch enthält, eine wässrige kolloidale Lösung oder eine Öl-in-Wasser-Dispersion des genannten Pulvers, eine Öl-in-Wasser-Dispersion von Zeacarotin oder Zeacarotingemisch oder eine öllösliche Dispersion von Zeacarotin oder Zeacarotingemisch in einem Triglycerid ist.

9. Farbstoffzusammensetzung nach Anspruch 8, die ein stabiles wasserlösliches oder -dispergierbares Pulver ist, wobei die Menge an Zeacarotin oder Zeacarotingemisch von etwa 0,1 bis etwa 30 Gew.-%, bezogen auf das Gesamtgewicht der Farbstoffzusammensetzung, beträgt.

10. Farbstoffzusammensetzung nach Anspruch 9, wobei Zeacarotin oder Zeacarotingemisch in einer Matrix oder einem Träger, vorzugsweise einer Matrix oder einem Träger, die ein oder mehrere Kohlenhydrate, ein modifiziertes Kohlenhydrat, ein Protein, ein modifiziertes Protein oder ein beliebiges Gemisch von zwei oder mehreren von diesen umfassen, fein dispergiert ist.

11. Farbstoffzusammensetzung nach Anspruch 10, wobei die Matrix oder der Träger, von denen mindestens ein Teil ein Schutzkolloid sein kann, aus einem Polysaccharidgummi, einer modifizierten Nahrungsstärke, einem Pektin, einem Maltodextrin, einem Protein, einem Ligninsulfonat und jedem beliebigen Gemisch dieser Substanzen ausgewählt ist.

12. Farbstoffzusammensetzung nach Anspruch 11, wobei die Matrix oder der Träger aus Natriumoctenylsuccinylstärke als modifizierter Stärke und einer Gelatine als Protein ausgewählt ist.

13. Farbstoffzusammensetzung nach einem der Ansprüche 7 bis 12, wobei die Zusammensetzung, je nachdem, ob sie in fester oder flüssiger Form vorliegt, (ferner) einen oder mehrere Excipienten und/oder Hilfsstoffe enthält, die aus einem oder mehreren von jeweils Mono-, Di-, Oligo- und Polysacchariden, Triglyceriden, wasserlöslichen Antioxidantien, fettlöslichen Antioxidantien und, nur im Fall fester Zusammensetzungen, zusätzlich einem Antiklumpmittel ausgewählt sind.

14. Farbstoffzusammensetzung nach Anspruch 13, wobei die Mono-, Di-, Oligo- und Polysaccharide aus Saccharose, Invertzucker, Glucose, Fructose, Lactose, Maltose, einem Zuckeralkohol, Stärke, einem Stärkehydrolysat und Glucosesirup ausgewählt sind.

15. Farbstoffzusammensetzung nach Anspruch 14, wobei das Stärkehydrolysat ein Dextrin oder Maltodextrin mit dem Bereich von 5-65 Dextroseäquivalenten ist und der Glucosesirup den Bereich von 20-95 Dextroseäquivalenten aufweist.

16. Farbstoffzusammensetzung nach einem der Ansprüche 8 und 13 bis 15, wobei das Triglycerid ein Pflanzenöl oder -fett, vorzugsweise Maisöl, Sonnenblumenöl, Sojabohnenöl, Distelöl, Rapsöl, Erdnussöl, Palmöl, Palmkernöl, Baumwollsamenöl oder Kokosnussöl, ist

17. Farbstoffzusammensetzung nach einem der Ansprüche 13 bis 16, wobei das wasserlösliche Antioxidans Ascorbinsäure oder ein Salz davon, vorzugsweise Natriumascorbat, ist und das fettlösliche Antioxidans ein Tocopherol, butyliertes Hydrotoluol; butyliertes Hydroxyanisol; Propylgallat; tert.-Butylhydroxychinolin oder ein Ascorbinsäureester einer Fettsäure, vorzugsweise Ascorbylpalmitat oder -stearat, ist.

18. Farbstoffzusammensetzung nach einem der Ansprüche 13 bis 17, wobei das Antiklumpmittel Kieselsäure ist.

19. Verfahren zur Herstellung einer Farbstoffzusammensetzung für Nahrungsmittelprodukte oder pharmazeutische Zusammensetzungen nach einem der Ansprüche 7 bis 8, **dadurch gekennzeichnet, dass** man in einer wässrigen oder kolloidalen Lösung aus einem Träger und gegebenenfalls einem oder mehreren wasserlöslichen Excipienten und/oder Hilfsstoffen eine Lösung oder Dispersion von ZeaCarotin oder Zeacarotingemisch und gegebenenfalls einem oder mehreren fettlöslichen Excipienten und/oder Hilfsstoffen in einem Triglycerid oder einem organischen Lösungsmittel oder in einem Gemisch von sowohl Triglycerid als auch organischem Lösungsmittel homogenisiert und, falls erforderlich, die so erhaltene Dispersion in eine feste Zusammensetzung umwandelt.

20. verfahren nach Anspruch 19, **dadurch gekennzeichnet, dass** man eine Lösung oder Dispersion von ZeaCarotin oder Zeacarotingemisch und einem öllöslichen Antioxidans in einem Triglycerid und gegebenenfalls einem organischen Lösungsmittel herstellt, die erhaltene Lösung oder Dispersion auf Ölbasis in einer wässrigen Lösung emulgiert, die aus einem Schutzhydrokolloid oder Träger, einem Kohlenhydrat und gegebenenfalls einem wasserlöslichen Antioxidans hergestellt wird, falls erforderlich, das organische Lösungsmittel aus der Emulsion entfernt, die Emulsion auf an sich bekannte Weise trocknet und schließlich die getrockneten Partikel abtrennt.

21. Verfahren zur Herstellung einer Farbstoffzusammensetzung für Nahrungsmittelprodukte oder pharmazeutische Zusammensetzungen nach einem der Ansprüche 7, 8 und 13 bis 17, die eine Suspension von fein verteiltem Zeacarotin oder Zeacarotingemisch in einem Triglycerid ist, **dadurch gekennzeichnet, dass** man Zeacarotin oder Zeacarotingemisch und gegebenenfalls einen oder mehreren fettlösliche Excipienten und/oder Hilfsstoffe in ein Triglycerid einbringt, eine ölmischbare Dispersion von Zeacarotin oder Zeacarotingemisch in dem Triglycerid bereitet und die Dispersion mahlt, z.B. mit einer Kugelmühle, wodurch die Dispersion in die gewünschte Suspension von fein verteiltem Zeacarotin oder Zeacarotingemisch in dem Triglycerid umgewandelt wird.

22. Nahrungsprodukt oder pharmazeutische zusammensetzung, insbesondere ein Getränk, ein Milchprodukt, ein Milchersatzprodukt, eine Süßigkeit, eine Fruchtzubereitung, ein Produkt auf Fettbasis, ein Produkt mit niedrigem Brennwert, eine Cerealie, ein Cerealienprodukt, ein Snack, eine Suppe, eine Soße, Gewürz, ein Tofuprodukt oder ein zusammengesetztes Nahrungsmittel, das eines oder mehrere der oben genannten Nahrungsmittelprodukte enthält, bzw. Tabletten oder Kapseln, **dadurch gekennzeichnet, dass** sie α-Zeacarotin oder β-zeacarotin oder ein Gemisch von α- und β-Zeacarotin in jedem beliebigen Verhältnis als hinzugefügten oder eingebrachten Farbstoff in einer Menge umfassen, die ausreicht, um dem Nahrungsmittelprodukt oder der pharmazeutischen Zusammensetzung eine gelbe bis grünlich-gelbe Farbe zu verleihen.

23. Nahrungsmittelprodukt nach Anspruch 22, wobei die Konzentration von Zeacarotin oder zeacarotingemisch, das als Farbstoff verwendet wird, von etwa 0,1 bis etwa 500 ppm, vorzugsweise von etwa 1 bis etwa 50 ppm, bezogen auf das Gesamtgewicht des Nahrungsmittelprodukts, beträgt.

## Revendications

1. Utilisation d'α-zéacarotène ou de β-zéacarotène ou d'un mélange d'α-zéacarotène et de β-zéacarotène en toute proportion relative en tant que colorant pour produits alimentaires ou compositions pharmaceutiques.

2. Utilisation selon la revendication 1, dans laquelle le colorant est l'α-zéacarotène.

3. Utilisation selon la revendication 1, dans laquelle le colorant est le β-zéacarotène.

4. Utilisation selon l'une quelconque des revendications 1 à 3, dans laquelle les produits alimentaires sont des boissons, des produits laitiers, des produits de substitution du lait, des produits sucrés, des préparations à base de fruits, des produits à base de matières grasses et des produits alimentaires faiblement calorifiques, des céréales et des produits de céréales, des collations, des soupes, des sauces, des assaisonnements, des produits de tofu et des produits composites contenant un ou plusieurs des produits alimentaires susmentionnés, et les compositions pharmaceutiques sont des comprimés ou des gélules.

5. Utilisation selon l'une quelconque des revendications 1 à 4, dans laquelle la concentration d'α-zéacarotène, de β-zéacarotène ou du mélange d'α-zéacarotène et β-zéacarotène utilisé en tant que colorant dans le produit alimentaire est comprise entre environ 0,1 et environ 500 ppm, de préférence entre environ 1 et environ 50 ppm, par rapport au poids total du produit alimentaire.

6. Utilisation selon l'une quelconque des revendications 1 à 5, dans laquelle l'α-zéacarotène, le β-zéacarotène ou le mélange d'α-zéacarotène et β-zéacarotène a été ajouté au produit alimentaire ou à la composition pharmaceutique à colorer, ou a été incorporé dans le produit alimentaire ou la composition pharmaceutique durant sa préparation, sous forme d'une composition colorante qui est une poudre stable soluble ou dispersible dans l'eau contenant le zéacarotène ou le mélange de zéacarotènes, une solution colloïdale aqueuse ou une dispersion huile dans l'eau de la poudre susmentionnée, une dispersion huile dans l'eau du zéacarotène ou du mélange de zéacarotènes ou une dispersion soluble dans l'huile du zéacarotène ou du mélange de zéacarotènes dans un triglycéride.

7. Composition colorante pour produits alimentaires ou compositions pharmaceutiques, **caractérisée en ce qu'**elle contient un α-zéacarotène, un β-zéacarotène ou un mélange d'α-zéacarotène et de β-zéacarotène en toute proportion relative en tant que colorant.

8. Composition colorante selon la revendication 7, qui est une poudre stable soluble ou dispersible dans l'eau contenant le zéacarotène ou le mélange de zéacarotènes, une solution aqueuse colloïdale ou une dispersion huile dans l'eau de la poudre susmentionnée, une dispersion huile dans l'eau du zéacarotène ou du mélange de zéacarotènes ou une dispersion soluble dans l'huile du zéacarotène ou du mélange de zéacaroténes dans un triglycéride.

9. Composition colorante selon la revendication 8, qui est une poudre stable soluble ou dispersible dans l'eau, la quantité de zéacarotène ou de mélange de zéacarotènes étant comprise entre environ 0,1 et environ 30 % en poids par rapport au poids total de la composition colorante.

10. Composition colorante selon la revendication 9, dans laquelle le zéacarotène ou le mélange de zéacarotènes est finement dispersé dans une matrice ou un véhicule, de préférence une matrice ou un véhicule comprenant un ou plusieurs éléments parmi un glucide, un glucide modifié, une protéine, une protéine modifiée ou un mélange de deux de ces éléments ou plus.

11. Composition colorante selon la revendication 10, dans laquelle la matrice ou le véhicule, dont au moins une partie peut être un colloïde protecteur, est choisi parmi une gomme de polysaccharide, un amidon alimentaire modifié, une pectine, une maltodextrine, une protéine, un sulfonate de lignine et tout mélange de ces substances.

12. Composition colorante selon la revendication 11, dans laquelle la matrice ou le véhicule est choisi parmi l'octényle succinate d'amidon sodique en tant qu'amidon alimentaire modifié et une gélatine en tant que protéine.

13. Composition colorante selon l'une quelconque des revendications 7 à 12, dans laquelle la composition, sous forme solide ou liquide selon le cas, contient (en outre) un ou plusieurs excipients et/ou adjuvants choisi parmi un ou plusieurs éléments parmi les monosaccharides, disaccharides, oligosaccharides et polysaccharides, les triglycérides, les antioxydants hydrosolubles, les antioxydants solubles dans les matières grasses et en outre, uniquement dans le cas de compositions solides, un agent antiagglomérant.

14. Composition colorante selon la revendication 13, dans laquelle les monosaccharides, disaccharides, oligosaccharides et polysaccharides sont choisis parmi le sucrose, le sucre inverti, le glucose, le fructose, le lactose, le maltose, un polyol, l'amidon, un hydrolysat d'amidon et le sirop de glucose.

15. Composition colorante selon la revendication 14, dans laquelle l'hydrolysat d'amidon est une dextrine ou une maltodextrine ayant de 5 à 65 équivalents en dextrose et le sirop de glucose a de 20 à 95 équivalents en dextrose.

16. Composition colorante selon l'une quelconque des revendications 8 et 13 à 15, dans laquelle le triglycéride est une matière grasse ou une huile végétale, de préférence l'huile de mais, l'huile de tournesol, l'huile de soja, l'huile de carthame, l'huile de colza, l'huile d'arachide, l'huile de palme, l'huile de palmiste, l'huile de graines de coton ou l'huile de noix de coco.

17. Composition colorante selon l'une quelconque des revendications 13 à 16, dans laquelle l'antioxydant hydrosoluble est l'acide ascorbique ou un de ses sels, de préférence l'ascorbate de sodium, et l'antioxydant soluble dans les matières grasses est un tocophérol, un hydroxytoluène butylé ; un hydroxyanisole butylé ; un gallate de propyle ; une tert-butyle hydroxyquinoléine ; ou un ester d'acide ascorbique d'un acide gras, de préférence le stéarate ou le palmitate ascorbyle.

18. Composition colorante selon l'une quelconque des revendications 13 à 17, dans laquelle l'agent antiagglomérant est l'acide silicique.

19. Procédé de préparation d'une composition colorante pour produits alimentaires ou compositions pharmaceutiques selon l'une quelconque des revendications 7 à 8, **caractérisé que** ce qu'il comprend l'homogénéisation dans une solution aqueuse ou colloïdale d'un véhicule et éventuellement d'un ou de plusieurs adjuvants et/ou excipients hydrosolubles, d'une solution ou d'une dispersion du zéacarotène ou du mélange de zéacarotènes et éventuellement d'un ou de plusieurs adjuvants et/ou excipients solubles dans les matières grasses dans un triglycéride ou un solvant organique, ou dans un mélange à la fois de triglycéride et d'un solvant organique, et, si nécessaire, la conversion de la dispersion ainsi obtenue en une composition solide.

20. Procédé selon la revendication 19, **caractérisé en ce qu'**il comprend la préparation d'une solution ou d'une dispersion du zéacarotène ou du mélange de zéacarotènes et d'un antioxydant soluble dans l'huile dans un triglycéride et éventuellement un solvant organique, l'émulsification de la solution ou dispersion à base d'huile résultante dans une solution aqueuse préparée à partir d'un hydrocolloïde protecteur ou d'un véhicule, d'un glucide et éventuellement d'un antioxydant hydrosoluble ; si nécessaire l'élimination du solvant organique de l'émulsion, le séchage de l'émulsion d'une manière connue en soi et enfin la séparation des particules séchées.

21. Procédé de préparation d'une composition de colorant pour produits alimentaires ou compositions pharmaceutiques selon l'une quelconque des revendications 7, 8 et 13 à 17 et qui est une suspension de zéacarotène ou d'un mélange de zéacarotènes finement divisé dans un triglycéride, **caractérisé en ce qu'**il comprend l'introduction du zéacarotène ou du mélange de zéacarotènes et éventuellement d'un ou de plusieurs adjuvants et/ou excipients solubles dans les matières grasses dans un triglycéride, l'obtention d'une dispersion miscible avec l'huile du zéacarotène ou du mélange de zéacarotènes dans le triglycéride, et le broyage de la dispersion, par exemple par broyage à boulets, transformant ainsi ladite dispersion en la suspension voulue de zéacarotène ou d'un mélange de zéacarotènes finement divisé dans le triglycéride.

22. Produit alimentaire ou composition pharmaceutique, notamment boisson, produit laitier, produit de substitution du lait, produit sucré, préparation à base de fruits, produit à base de matières grasses, produit alimentaire faiblement calorifique, céréales, produit de céréales, collation, soupe, sauce, assaisonnement, produit de tofu ou produit composite contenant un ou plusieurs des produits alimentaires susmentionnés, ou respectivement comprimés ou gélules, **caractérisés en ce qu'**ils comprennent de l'α-zéacarotène ou du β-zéacarotène ou un mélange d'α- et de β-zéacarotène en toute proportion relative en tant que colorant ajouté ou incorporé en une quantité suffisante pour conférer une couleur jaune à vert-jaune au produit alimentaire ou à la composition pharmaceutique.

23. Produit alimentaire selon la revendication 22, dans lequel la concentration de zéacarotène ou du mélange de zéacarotènes utilisé en tant que colorant est comprise entre environ 0,1 et environ 500 ppm, de préférence entre environ 1 et environ 50 ppm, par rapport au poids total du produit alimentaire.
